(19) Europäisches Patentamt
European Patent Office
Office européen des brevets

(11) Veröffentlichungsnummer: **0 477 611 A1**

(12) **EUROPÄISCHE PATENTANMELDUNG**

(21) Anmeldenummer: **91114905.2**

(22) Anmeldetag: **04.09.91**

(51) Int. Cl.5: **C07D 487/22**, C07D 207/335, //(C07D487/22,259:00,209:00, 209:00,209:00,209:00)

(30) Priorität: **20.09.90 DE 4029768**
**20.03.91 DE 4109085**

(43) Veröffentlichungstag der Anmeldung:
**01.04.92 Patentblatt 92/14**

(84) Benannte Vertragsstaaten:
**CH DE FR GB IT LI**

(71) Anmelder: **BASF Aktiengesellschaft**
**Carl-Bosch-Strasse 38**
**W-6700 Ludwigshafen(DE)**

(72) Erfinder: **Franck, Burchard, Prof. Dr.**
**Coesfeldweg 41**
**W-4400 Muenster(DE)**
Erfinder: **Koenig, Hartmann, Dr.**
**Albert-Einstein-Allee 16**
**W-6703 Limburgerhof(DE)**
Erfinder: **Eickmeier, Christian**
**Westerkoetterstrasse 142 a**
**W-4400 Muenster(DE)**
Erfinder: **Voelker, Michael**
**Stadtlohnweg 33**
**W-4400 Muenster(DE)**
Erfinder: **Wessel, Thomas**
**Pastorskamp 31**
**W-4405 Nottuln 2(DE)**

(54) **Azaporphyrinderivate sowie Biladienderivate als deren Zwischenprodukte.**

(57) Azaporphyrinderivate der Formel

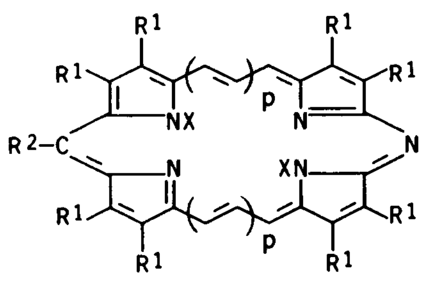

in der

R1 und R2 jeweils Wasserstoff, gegebenenfalls substituiertes $C_1$-$C_{20}$-Alkyl, $C_5$-$C_7$-Cycloalkyl oder gegebenenfalls substituiertes Phenyl oder zwei benachbarte Reste $R^1$ zusammen mit den Kohlenstoffatomen, an die sie gebunden sind, einen ungesättigten oder aromatischen 5- bis 7-gliedrigen carbocyclischen Ring,

X jeweils Wasserstoff oder $C_1$-$C_4$-Alkyl und

p jeweils 1 oder 2 bedeuten,

Biladiene der Formel



Rank Xerox (UK) Business Services

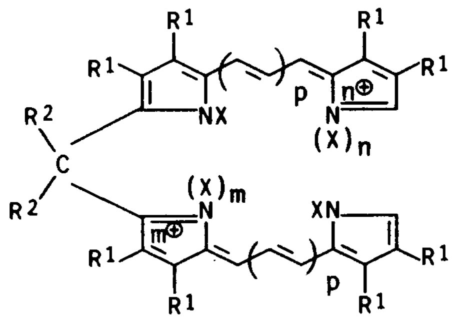

(m+n) $An^{\ominus}$ ,

in der $R^1$, $R^2$, X und p jeweils die obengenannte Bedeutung besitzen, m und n jeweils 0 oder 1 und $An^{\ominus}$ ein Anion bedeuten, als deren Zwischenprodukte sowie ihre Verwendung zur Herstellung von vinylogen Porphyrinderivaten.

Die vorliegende Erfindung betrifft neue Azaporphyrinderivate der Formel I

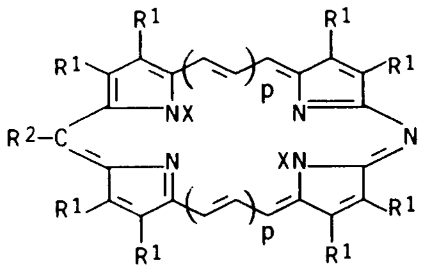

(I),

in der

die Reste $R^1$ und $R^2$ unabhängig voneinander jeweils Wasserstoff, gegebenenfalls substituiertes $C_1$-$C_{20}$-Alkyl, $C_5$-$C_7$-Cycloalkyl oder gegebenenfalls substituiertes Phenyl oder zwei benachbarte Reste $R^1$ zusammen mit den Kohlenstoffatomen, an die sie gebunden sind, einen ungesättigten oder aromatischen 5- bis 7-gliedrigen carbocyclischen Ring,

die Reste X unabhängig voneinander jeweils Wasserstoff oder $C_1$-$C_4$-Alkyl und

p unabhängig voneinander jeweils 1 oder 2 bedeuten.

Die vorliegende Erfindung betrifft weiterhin neue Biladiene der Formel II

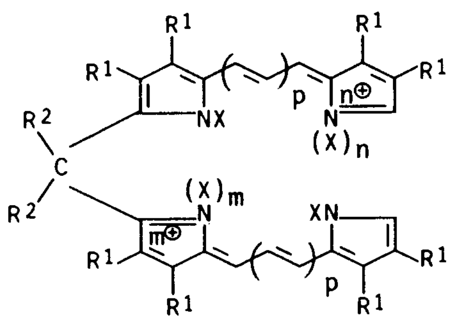

$(m+n)\ An^{\ominus}$ (II),

in der

die Reste $R^1$ und $R^2$ unabhängig voneinander jeweils Wasserstoff, gegebenenfalls substituiertes $C_1$-$C_{20}$-Alkyl, $C_5$-$C_7$-Cycloalkyl oder gegebenenfalls substituiertes Phenyl oder zwei benachbarte Reste $R^1$ zusammen mit den Kohlenstoffatomen, an die sie gebunden sind, einen ungesättigten oder aromatischen 5- bis 7-gliedrigen carbocyclischen Ring,

die Reste X unabhängig voneinander jeweils Wasserstoff oder $C_1$-$C_4$-Alkyl,

m und n unabhängig voneinander jeweils 0 oder 1,

p unabhängig voneinander jeweils 1 oder 2 und

$An^{\ominus}$ ein Anion bedeuten.

Aus der US-A-4 798 891 sind Porphyrinderivate bekannt, die außer den vier Pyrrolstickstoffatomen keine weiteren Heteroatome im Porphyrinring aufweisen.

Aus Ann. Chem. 1976, Seiten 1637 bis 1658, sind weiterhin bereits Biladiene bekannt. Es hat sich jedoch gezeigt, daß die dort beschriebenen Komponenten für die Herstellung von vinylogen Porphyrinverbindungen nicht geeignet sind.

Aufgabe der vorliegenden Erfindung war es nun, neue Porphyrinderivate bereitzustellen, die über ein zusätzliches Stickstoffatom im Porphyrinring verfügen.

Eine weitere Aufgabe der vorliegenden Erfindung war es, neue Biladienderivate bereitzustellen, mittels derer die Synthese von vinylogen Porphyrinverbindungen und Azaporphyrinderivaten in vorteilhafter Weise vorgenommen werden kann.

Demgemäß wurden die eingangs näher bezeichneten Azaporphyrinderivate der Formel I sowie die Biladienderivate der Formel II gefunden.

Alle in den obengenannten Formeln I und II auftretenden Alkyl- und Alkylengruppen können sowohl geradkettig als auch verzweigt sein.

Wenn in den obengenannten Formeln I und II substituierte Alkylgruppen auftreten, so können als

Substituenten z.B. Phenyl oder $C_1$-$C_{12}$-Alkoxycarbonyl in Betracht kommen.

Wenn in den obengenannten Formeln I und II substituierte Phenylgruppen auftreten, so können als Substituenten z.B. Halogen, dabei insbesondere Chlor oder Brom, Cyano, $C_1$-$C_4$-Alkyl, $C_1$-$C_4$-Alkoxy, Nitro oder $C_1$-$C_4$-Alkoxycarbonyl in Betracht kommen.

Reste $R^1$ und $R^2$ sind z.B. Methyl, Ethyl, Propyl, Isopropyl, Butyl, Isobutyl, sec-Butyl, tert-Butyl, Pentyl, Isopentyl, Neopentyl, tert-Pentyl, Hexyl, 2-Methylpentyl, Heptyl, 1-Ethylpentyl, Octyl, 2-Ethylhexyl, Isooctyl, Nonyl, Isononyl, Decyl, Isodecyl, Undecyl, Dodecyl, Tridecyl, Isotridecyl, Tetradecyl, Pentadecyl, Hexadecyl, Heptadecyl, Octadecyl, Nonadecyl, Eicosyl (Die obigen Bezeichnungen Isooctyl, Isononyl, Isodecyl und Isotridecyl sind Trivialbezeichnungen und stammen von den nach der Oxosynthese erhaltenenen Alkoholen - vgl. dazu Ullmanns Encyklopädie der technischen Chemie, 4. Auflage, Band 7, Seiten 215 bis 217, sowie Band 11, Seiten 435 und 436.), Benzyl, 1- oder 2-Phenylethyl, Methoxycarbonylmethyl, Ethoxycarbonylmethyl, Propoxycarbonylmethyl, Isopropoxycarbonylmethyl, Butoxycarbonylmethyl, Isobutoxycarbonylmethyl, sec-Butoxycarbonylmethyl, 2-Methoxycarbonylethyl, 2-Ethoxycarbonylethyl, 2-Propoxycarbonylethyl, 2-Isopropoxycarbonylethyl, 2-Butoxycarbonylethyl, 2- oder 3-Methoxycarbonylpropyl, 2- oder 3-Ethoxycarbonylpropyl, 2- oder 4-Methoxycarbonylbutyl, 2- oder 4-Ethoxycarbonylbutyl, Cyclopentyl, Cyclohexyl, Methylcyclohexyl, Cyloheptyl, Phenyl, 2-, 3- oder 4-Chlorphenyl, 2-, 3- oder 4-Bromphenyl, 2,4-Dichlorphenyl, 2-, 3- oder 4-Cyanophenyl, 2-, 3- oder 4-Methylphenyl, 2,4-Dimethylphenyl, 2-, 3- oder 4-Methoxyphenyl oder 2,4-Dimethoxyphenyl.

Wenn zwei benachbarte Reste $R^1$ zusammen mit den Kohlenstoffatomen, an die sie gebunden sind, einen ungesättigten oder aromatischen 5- bis 7-gliedrigen carbocyclischen Ring bedeuten, so kann dabei beispielsweise der Cyclopenten-, Cyclopentadien-, Cyclohexen-, Cyclohexadien-, Benzol-, Cyclohepten-, Cycloheptadien- oder Cycloheptatrienring in Betracht kommen. Die genannten Ringe können jeweils noch durch $C_1$-$C_4$-Alkyl substituiert sein.

$An^{\ominus}$ in Formel I bedeutet ein Anion. Dabei kommen sowohl anorganische als auch organische Anionen in Betracht. Geeignete Anionen sind z.B. Halogenid, wie Chlorid, Bromid oder Iodid, Sulfat, Perchlorat, Phosphat, Tetrafluoroborat, Trichlorozinkat, Methansulfonat, Benzolsulfonat, 4-Methylphenylsulfonat, Acetat, Lactat, Salicylat oder Tetraphenylborat.

Bevorzugt sind Azaporphyrinderivate der Formel I, in der die Reste $R^1$ und $R^2$ unabhängig voneinander jeweils Wasserstoff, gegebenenfalls durch Phenyl oder $C_1$-$C_4$-Alkoxycarbonyl substituiertes $C_1$-$C_4$-Alkyl oder Phenyl und die Reste X unabhängig voneinander jeweils Wasserstoff, Methyl oder Ethyl bedeuten.

Bevorzugt sind ebenfalls Biladiene der Formel II, in der die Reste $R^1$ und $R^2$ unabhängig voneinander jeweils Wasserstoff, gegebenenfalls durch Phenyl oder $C_1$-$C_4$-Alkoxycarbonyl substituiertes $C_1$-$C_4$-Alkyl oder Phenyl und die Reste X unabhängig voneinander jeweils Wasserstoff, Methyl oder Ethyl bedeuten und m, n, p und $An^{\ominus}$ jeweils die obengenannte Bedeutung besitzen.

Die neuen Azaphorphyrinderivate der Formel können durch Umsetzung der Biladienderivate der Formel II mit Ammoniak und anschließender Oxidation erhalten werden.

Die neuen Biladiene der Formel II können nach an sich bekannten Methoden erhalten werden. Beispielsweise kann man Dipyrrylmethane der Formel IV

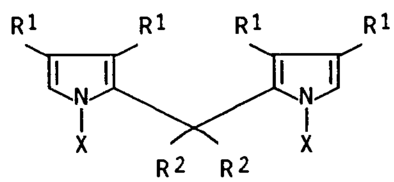

(IV),

in der $R^1$, $R^2$ und X jeweils die obengenannte Bedeutung besitzen, mit Aldehyden der Formel V

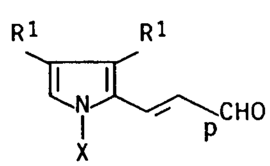

(V),

in der $R^1$, X und p jeweils die obengenannte Bedeutung besitzen, in Gegenwart eines sauren Kondensationsmittels, z.B. Bromwasserstoffsäure, umsetzen.

Die Dipyrrylmethane der Formel IV sowie die Aldehyde der Formel V sind an sich bekannt oder können

nach an sich bekannten Methoden erhalten werden, wie sie z.B. in J. Org. Chem., Band 41, Seiten 2826 bis 2835, 1976 sowie in der US-A-4 798 891 beschrieben sind.

Bei den erfindungsgemäßen Biladienen der Formel II handelt es sich um wertvolle Zwischenprodukte für die Synthese von vinylogen Porphyrinderivaten der Formel III

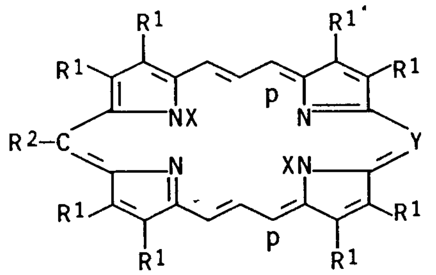

(III),

in der Y für Stickstoff oder den Rest $CR^2$ steht und $R^1$, $R^2$, X und p jeweils die obengenannte Bedeutung besitzen.

Die Verbindungen der Formel III (mit Y = $CR^2$) sind aus der US-A-4 798 891 bekannt.

Die Biladiene der Formel II können durch Umsetzung mit Aldehyd der Formel VI

$R^2$-CHO (VI),

in der $R^2$ die obengenannte Bedeutung besitzt, in Gegenwart eines sauren Kondensationsmittels, z.B. Bromwasserstoffsäure, und anschließender Oxidation in die Porphyrine III (mit Y = $CR^2$) übergeführt werden.

Die erfindungsgemäßen Azaporphyrine der Formel I können z.B. als Sensibilisatoren für photochemische Reaktionen, in optischen Speichern oder in der Photomedizin, z.B. in der photodynamischen Tumortherapie Anwendung finden.

Die folgenden Beispiele sollen die Erfindung näher erläutern.

Beispiel 1

Man löste 355 mg (2,00 mmol) 3-(3,4-Diethylpyrr-2-yl)acrolein und 258 mg (1,00 mmol) Bis(3,4-Diethylpyrr-2-yl)methan in 7 ml Methanol und kühlte auf -15 °C. Unter intensivem Rühren wurden bei dieser Temperatur 0,4 ml 62 gew.-%ige Bromwasserstoffsäure zugetropft und anschließend 5 Minuten weitergerührt. Man filtrierte den tiefgrünen Niederschlag ab und wusch mit wenig eiskaltem Methanol sowie mit n-Hexan. Durch Trocknen im Hochvakuum bei 40 °C erhielt man 601 mg (81 %) des Biladiens der Formel

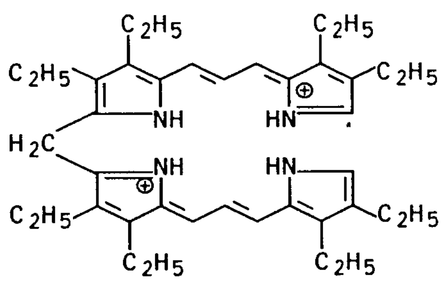

2Br⊖

in Form dunkelgrüner, feiner Nadeln. Schmp.: Zers. ohne Schmelzen ab ca. 160 °C.

$^1H$ NMR (300 MHz, $CD_2Cl_2$): δ = 0,75 (t, J = 7,4 Hz, 6 H, $CH_2CH_3$), 1,15-1,23 (m, 18 H, $CH_2CH_3$), 2,46-2,57, 2,59-2,71 (2m, je 8 H, $CH_2CH_3$), 4,62 (s, 2 H, Brücken-$CH_2$), 7,43-7,50 (m, 6 H, CH=CHCH u. Pyrrol-α-H), 9,03 (t,J = 13,6 Hz, 2 H, CH=CHCH), 11,80, 12,84 (2 br. s, je 2 H, NH).

MS (70 eV): m/z (%) = 578 (20) [M - 2 Br], 577 (70) [M - 2 Br - H], 576 (27) [M - HBr] 547 (6) [576 - $C_2H_5$], 453 (6) [576 - Monopyrroleinheit].

IR (KBr): $\bar{\nu}$ = 2960, 2920, 2860 $cm^{-1}$ (w, CH), 1570 $cm^{-1}$ (s, konjug. C=C).

UV/VIS ($CHCl_3$): $\lambda_{max}$ (lg ε) = 657 nm (5,267), 547 nm (4,664), 504 nm (4.390).

| | | | | |
|---|---|---|---|---|
| $C_{39}H_{54}Br_2N_4$ (738.7) Ber. | C 63,41 | H 7,37 | N 7,58 | Br 21,63 |
| Gef. | C 63,30 | H 7,50 | N 7,56 | Br 21,53 |

Beispiel 2

Man löste 280 mg (1,37 mmol) 5-(3,4-Diethylpyrrol-2-yl)penta-2,4-dienal und 180 mg (0,69 mmol) Bis-(3,4-diethylpyrrol-2-yl)-methan in 7 ml Methanol und kühlte auf -15 °C. Unter intensivem Rühren wurden bei dieser Temperatur 0,3 ml 62 gew.-%ige Bromwasserstoffsäure zugetropft und anschließend 10 Minuten weitergerührt. Man saugte den olivgrünen Niederschlag ab und wusch mit wenig eiskaltem Methanol sowie n-Pentan. Aus der Mutterlauge kristallisierte im Tiefkühlschrank bei -30 °C weiteres Produkt. Durch Trocknen im Ölpumpenvakuum erhielt man das analysenreine Biladiensalz der Formel

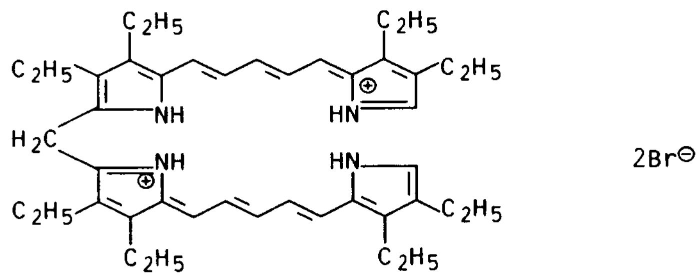

als dunkelgrünes Pulver, das in Lösung eine tiefblaue Farbe aufwies.
Ausb.: 463 mg (40,3 %)
Schmp.: Zers. ohne Schmelzen ab ca. 120 °C
DC (Laufmittelsystem Dichlormethan/Methanol 20:1 v/v): $R_f$ = 0,60
$^1H$ NMR (300 MHz, [$D_6$]DMSO): $\delta$ = 1,02-1,21 (m, 24 H, $CH_2CH_3$), 2,33-2,41, 2,52-2,60 (2 m, je 8 H, $CH_2CH_3$), 5,56, 5,61 (2 br. s, 2 H, Brücken-$CH_2$), 6,75-7,27 (m, 12 H, CH=CH-CH=CH-CH u. Pyrrol-α-H), 11,06, 11,18 (2 br. s, je 2 H, NH).
MS (70 eV): m/z (%) = 629 (20) [M - 2 Br - H], 628 (24) [M - 2 HBr], 599 (6) [628 - $C_2H_5$), 491 (30) [628 - Monopyrroleinheit].
IR (KBr): $\nu$ = 2980, 2910, 2860 $cm^{-1}$ (w, CH), 1570 (s. konj. C=C).
UV/VIS ($CH_2Cl_2$): $\lambda_{max}$ (lg $\epsilon$) = 785 nm (4,340), 621 (5,544), 410 (4,237).

| | | | | |
|---|---|---|---|---|
| $C_{43}H_{58}Br_2N_4$ (790,8) Ber. | C 62,31 | H 7,39 | N 7,09 | Br 20,21 |
| Gef. | C 65,30 | H 7,47 | N 7,06 | Br 20,22 |

Beispiel 3

0,25 mmol des Biladiensalzes der Formel

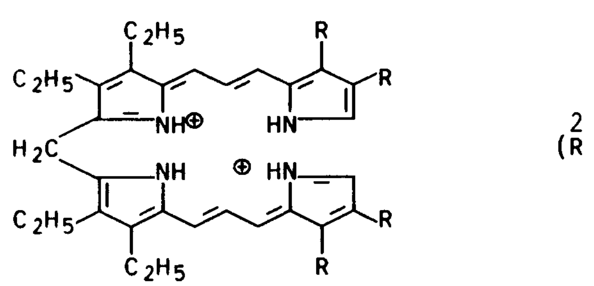

(R = Methyl)

wurden in 90 ml Methanol gelöst. Die tiefblaue Lösung versetzte man mit 3,5 ml 25 gew.%iger wäßriger

Ammoniaklösung. Zu der nunmehr gelbgrünen Lösung gab man 284 mg (1,25 mmol) 2,3-Dichlor-5,6-dicyano-1,4-benzochinon,gelöst in 15 ml Methanol, und rührte 1 Stunde bei Raumtemperatur.

Anschließend wurde 1 Stunde im Eisbad stehen gelassen, um die Fällung zu vervollständigen, und dann filtriert. Der Filterrückstand wurde in Dichlormethan/Methanol (20:1 v/v) aufgenommen, zusammen mit dem unlöslichen Rückstand auf eine Kieselgelsäule gegeben und säulenchromatographiert (Merck-Kieselgel 60, Korngröße 0,063-0,200 mm, Laufmittelsystem Dichlormethan/Methanol 20:1 v/v). Durch Eindampfen der grünen Eluatfraktionen unter vermindertem Druck erhielt man ein Azaporphyrin der Formel

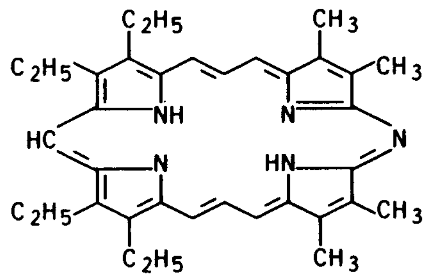

als blaugrünen, glänzenden Farblack in analysenreiner Form.
Ausbeute: 23 mg (17 %); Zersetzungspunkt: 320-330 °C.
FT-IR (KBr): $\bar{\nu}$ = 3437 $cm^{-1}$ (s, br., NH), 3034 (w, =CH), 2966, 2928, 2870 (s, CH).
$^1$H-NMR (300 MHz, $CDCl_3$ + 1 % FTA-$d_1$): $\delta$ = -7,47 (t, J = 13,7 Hz, 2 H, Trimethin-Brücken-CH=CHCH), 2,11 (t, J = 7.5 Hz, 12H, $CH_2CH_3$), 3,93 (s, 6H, $CH_3$), 3,97- (s, 6H, $CH_3$), 4,48 (q, J = 7,5 Hz, 8H, $CH_2CH_3$), 11,36 (s, 1H, Monomethin-Brücken-CH), 11,92, 11,99 (d, J = 13,2 Hz, 2H; d, J = 14,3 Hz, 2H; Trimethin-Brücken-CH=CHCH).
MS (70 eV): m/z (%) = 532 (22) [$M^+$ + H], 531 (58) [$M^+$], 516 (19) [$M^+$ - $CH_3$], 502 (21) [$M^+$ - $C_2H_5$], 487 (15) [516 - $C_2H_5$], 473 (17) [502 - $C_2H_5$], 458 (16) [473 - $CH_3$], 266 (42) [$M^+$/2].
UV/VIS ($CH_2Cl_2$): $\lambda_{max}$ (lg $\epsilon$) = 451 nm (5,129), 589 (3,898), 630 (4,037), 692 (3,672), 775 (3,393).
UV/VIS ($CH_2Cl_2$ + 1 % TFA): $\lambda_{max}$ (lg $\epsilon$) = 451 nm (5,523), 612 (3,937), 659 (4,097), 681 (4,157), 741 (3,393).

| | |
|---|---|
| $C_{35}H_{41}N_5$ Ber. | 531,3362 |
| Gef. | 531,3371 (MS) |

| | | | |
|---|---|---|---|
| $C_{35}H_{41}N_5 \cdot 0,3\ CH_2Cl_2$ (557,22) Ber. | C 76,09 | H 7,52 | N 12,57 |
| Gef. | C 76,43 | H 7,83 | N 12,41 |

Beispiel 4

Beispiel 4 wurde analog Beispiel 3 durchgeführt, jedoch wurde als Biladiensalz die entsprechende Ethylverbindung (R = Ethyl) verwendet.

Man erhielt 29 mg (20 %) der Verbindung der Formel

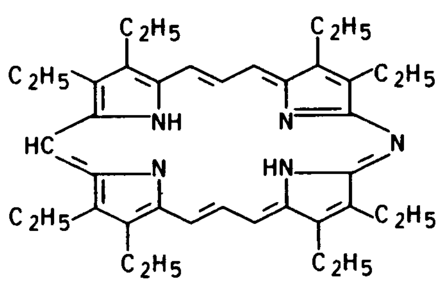

als blaugrünen, glänzenden Farblack. Zersetzungspunkt: 320-330 °C.
FT-IR (KBr): $\bar{\nu}$ = 3431 $cm^{-1}$ (s, br., NH), 3038 (w, =CH), 2963, 2930, 2869 (s, CH).
$^1$H-NMR (300 MHz, $CDCl_3$ + 1 % TFA-$d_1$): $\delta$ = -7,55 (t, J = 13,7 Hz, 2 H, Trimethin-Brücken-CH=CHCH), 2,12 ($m_c$, 24H, $CH_2CH_3$), 4,50 ($m_c$, 16H, $CH_2CH_3$), 11,40 (s, 1H, Monomethin-Brücken-CH), 11,96, 12,03 (d, J = 13,2 Hz, 2H; d, J = 14,3 Hz, 2H; Trimethin-Brücken-CH=CHCH).
MS (70 eV): m/z (%) = 587 (22) [$M^+$], 500 (18) [$M^+$ - $3C_2H_5$], 293 (26) [$M^+$/2], 280 (27) [Dipyrrotrimethin-Einheit$^+$], 249 (27) [$M^+$/2 - $C_2H_5$ - $CH_3$], 235 (34) [$M^+$/2 - $2C_2H_5$].
UV/VIS ($CH_2Cl_2$): $\lambda_{max}$ (lg $\epsilon$) = 450 nm (5,186), 590 (4,076), 631 (4,204), 694 (3,787), 776 (3,611).
UV/VIS ($CH_2Cl_2$ + 1 % TFA): $\lambda_{max}$ (lg $\epsilon$) = 453 nm (5,568), 614 (4,084), 660 (4,207), 682 (4,222), 743 (3,586).

| | |
|---|---|
| $C_{39}H_{49}N_5$ Ber. | 587,3988 |
| Gef. | 587,4000 (MS) |

| | | | |
|---|---|---|---|
| $C_{39}H_{49}N_5 \cdot 0,1\ CH_2Cl_2$ (596,34) Ber. | C 78,75 | H 8,32 | N 11,74 |
| Gef. | C 78,81 | H 8,77 | N 10,85 |

Beispiel 5

79,5 mg (0,10 mmol) des Biladiensalzes aus Beispiel 2 wurden in 30 ml Methanol gelöst. Die tiefblaue Lösung versetzte man mit 2,0 ml konz. wäßriger Ammoniaklösung. Zu dieser Lösung gab man nach 10 Minuten 114 mg (0,50 mmol) Dichlordicyanochinon in 6 ml Methanol und rührte 1 Stunde bei Raumtemperatur. Anschließend wurde 1 Stunde im Tiefkühlschrank bei -30 °C stehen gelassen und sodann der grünliche Niederschlag abgesaugt. Der Filterrückstand wurde so weit wie möglich in Dichlormethan/Methanol (20:1 v/v) aufgenommen und zusammen mit unlöslichem Rückstand über eine kurze Säule chromatographiert (Laufmittelsystem: Dichlormethan/Methanol 20:1 v/v, Säulendurchmesser: 5 cm, Füllhöhe: 15 cm). Durch Eindampfen der roten Eluatfraktionen unter vermindertem Druck erhielt man das Azaporphyrin der Formel

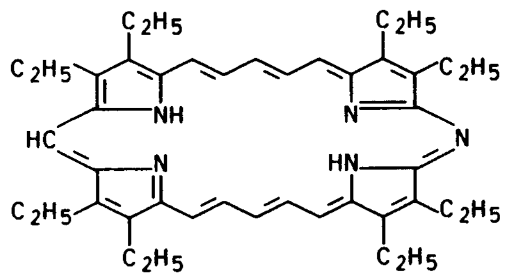

als blaugrünen, glänzenden Farblack.
Ausb.: 11,4 mg (17,9 %)
Schmp.: Zers. ab ca. 215 °C
DC (Laufmittelsystem: Dichlormethan/Methanol 20:1 v/v): $R_f$ = 0,23
$^1$H NMR (300 MHz, $CF_3CO_2D$): $\delta$ = -4,47 ($m_c$, 4 H, CHCHCHCHCH), 2,15 ($m_c$, 24 H, $\beta$-$CH_3$), 4,46 ($m_c$, 16 H, $\beta$-$CH_2$), 11,26 (s, 1 H, Monomethin-Brücken-H), 11,77, 12,03 (2 d, J = 12,5 Hz, 4 H, CHCHCHCHCH), 12,53 (t, J = 12,7 Hz, 2 H, CHCHCHCHCH).
MS (70 eV): m/z (%) = 641 (24) [M + 2], 640 (32) [M + 1], 639 (56) [$M^+$], 624 (24) [M - $CH_3$], 610 (40) [M - $C_2H_5$], 320 (30) [M + 1)/2].
IR (KBr): $\nu$ = 3410 $cm^{-1}$ (w, NH), 2960, 2920, 2860 (s, CH).
UV/VIS ($CH_2Cl_2$): $\lambda_{max}$ (lg $\epsilon$) = 490 nm (5,229), 649 (4,445), 697 (4,181).
UV/VIS ($CH_2Cl_2$ + 1 % TFA): $\lambda_{max}$ (lg $\epsilon$) = = 503 nm (5,560), 681 (4,513), 735 (4,440).

$C_{43}H_{53}N_5$ Ber. 639,4300 Gef. 639,4295 (MS)

| | | | |
|---|---|---|---|
| Ber. | C 80,71 | H 8,35 | N 10,94 |
| Gef. | C 80,65 | H 8,41 | N 10,85 |

Beispiel 6

73,9 mg (100 µmol) des Biladiens aus Beispiel 1 wurden in 40 ml Methanol gelöst und mit 10 ml 36 gew.-%iger wäßriger Formaldehydlösung versetzt. Nach Zugabe von 0,5 ml 5 gew.-%iger Bromwasserstoffsäure erhitzte man 30 Minuten unter Rückfluß zum Sieden. Anschließend wurde das Reaktionsgemisch rasch auf 20 °C abgekühlt, mit einer Lösung von 45 mg (0,2 mmol) Dichlordicyanochinon in 5 ml Dichlormethan versetzt und 2 Stunden gerührt. Man verteilte zwischen Dichlormethan und 2 N Sodalösung, trennte die wäßrige Phase ab und extrahierte mit Dichlormethan. Die vereinigten organischen Phasen wurden mit Wasser gewaschen und über Magnesiumsulfat getrocknet. Nach weitgehendem Abdampfen des Lösungsmittels reinigte man durch Säulenchromatographie an Kieselgel (Laufmittelsystem: Dichlormethan/Methanol 20:1 (v/v). Durch Eindampfen der grünen Eluatfraktionen unter vermindertem Druck erhielt man 24,4 mg (42 %) des Porphyrins der Formel

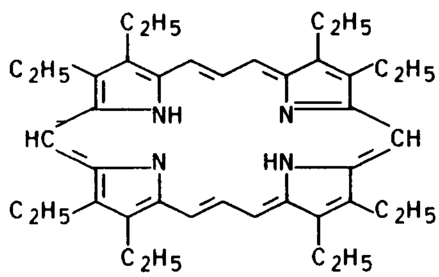

als blaugrünen, glänzenden Farblack. Umkristallisation aus Chloroform/Ether lieferte feine, tiefgrüne Nadeln.
Schmp.: Zers. ab ca. 280 °C (bei raschem Erhitzen).
$^1$H NMR (300 MHz, $CDCl_3$): $\delta$ = -8,32 (t, J = 13,3 Hz, 2 H, Trimethin-Brücken-CH = CHCH), -4,73 (br. s, 2 H, NH), 2,05-2,17 (m, 24 H, $\beta$-$CH_3$), 4,30-4,49 (m, 16 H, $\beta$-$CH_2$), 10,48 (s, 2 H, Monomethin-Brücken-CH), 11,88 (d, J = 13,3 Hz, 4 H, Trimethin-Brücken-CH = CHCH).
$^1$H NMR (300 MHz, $CDCl_3$ + 1 % TFA): $\delta$ = -9,54 (t, J = 13,9 Hz, 2 H, Trimethin-Brücken-CH = CHCH), -6,61 (br. s, 4 H, NH), 2,31 ($m_c$, 24 H, $\beta$-$CH_3$), 4,85 ($m_c$, 16 H, $\beta$-$CH_2$), 11,96 (s, 2 H, Monomethin-Brücken-CH), 12,78 (d, J = 13,9 Hz, 4 H, Trimethin-Brücken-CH = CHCH).
MS (70 eV): m/z (%) = 587 (34) [M + 1], 586 (42) [$M^{+}$], 571 (12) [M - $CH_3$], 557 (23) [M - $C_2H_5$], 513 (10) [M - 2 $C_2H_5$ - $CH_3$].
FT-IR (KBr): $\bar{\nu}$ = 3034 $cm^{-1}$ (w, =CH), 2963, 2928, 2868 $cm^{-1}$ (m, CH), 1659, 1597 $cm^{-1}$ (w, konjug. C=C), 1055, 1013, 953 $cm^{-1}$ (s).
UV/VIS ($CH_2Cl_2$): $\lambda_{max}$ (lg $\epsilon$) = 463 nm (5,622), 489 nm (4,710), 592 nm (4,035).
UV/VIS ($CH_2Cl_2$ + 1 % TFA): $\lambda_{max}$ (lg $\epsilon$) = 434 nm (4,770), 460 nm (6,049), 607 nm (4,097), 621 nm (4,310), 678 nm (4,111), 691 nm (3,901).

| | | | |
|---|---|---|---|
| $C_{40}H_{50}N_4$ • 0,07 $CHCl_3$ (595,2) Ber. | C 80,86 | H 8,48 | N 9,41 |
| Gef. | C 80,91 | H 8,57 | N 9,45 |

Beispiel 7

40,0 mg (50,6 µmol) des Biladiensalzes aus Beispiel 2 wurden in 100 ml Methanol gelöst und mit 2 ml 5 gew.-%iger Bromwasserstoffsäure versetzt. Nach Zugabe von 10 ml 36 gew.-%iger wäßriger Formaldehyd-Lösung erhitzte man die Lösung 20 Minuten auf 70 °C unter Argonatmosphäre. Anschließend wurde das Reaktionsgemisch rasch auf 20 °C abgekühlt, mit einer Lösung von 22 mg (0,1 mmol) Dichlordicyanochinon in 10 ml Dichlormethan versetzt und 1,5 Stunden unter Lichtausschluß gerührt. Man

verteilte zwischen Dichlormethan und 2 N Natriumcarbonatlösung, trennte die wäßrige Phase ab und extrahierte mit Dichlormethan. Die vereinigten organischen Phasen wurden mit Wasser gewaschen und über Magnesiumsulfat getrocknet. Nach Abdampfen des Lösungsmittels reinigte man durch zweimalige Flash-Chromatographie (Säulendurchmesser: 6 cm, Füllhöhe: 30 cm, Laufmittelsystem: Dichlormethan/Aceton/Methanol 8:3:1 v/v/v). Durch Eindampfen der roten Eluatfraktionen und anschließendes Trocknen im Ölpumpenvakuum erhielt man das Porhydrin der Formel

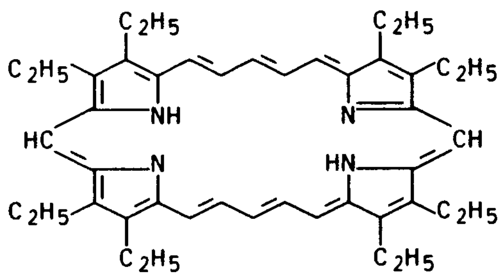

als blaugrünen, glänzenden Farblack (in der Durchsicht rotviolett).
Ausb.: 13,8 mg (42,7 %)
Schm.: Zers. ab ca. 180 ° C (bei raschem Erhitzen).
DC (Laufmittelsystem $CH_2Cl_2$/Aceton/Methanol 8:3:1 v/v/v): $R_f$ = 0,25
$^1H$ NMR (300 MHz, [$D_6$]DMSO + 1 % TFA): $\delta$ = -9,79 (br. s, 4 H, CHCHCHCHCH), -5,77 (br. s, 4 H, NH), 2,22, 2,34 (2 t, J = 7,5 Hz, je 12 H, $\beta$-$CH_3$), 5.03 ($m_c$, 16 H, $\beta$-$CH_2$), 12,26 (s, 2H, Monomethin-Brücken-CH), 13,51 (d, J = 13,8 Hz, 4 H, CHCHCHCHCH), 14,35 (t, J = 7,4 Hz, 2 H, CHCHCHCHCH).
MS (70 eV): m/z (%) = 639 (20) [M + 1], 638 (32) [$M^+$], 609 (18) [M - $C_2H_5$], 319 (12) [1/2 M].
IR (KBr): $\nu$ = 2980, 2920, 2910 $cm^{-1}$ (m, CH), 1590 (w, konj. C = C).
UV/VIS ($CH_2Cl_2$): $\lambda_{max}$ (lg $\epsilon$) = 507 nm (5,435), 528 (5,232), 553 (4,927), 663 (4,393).
UV/VIS ($CH_2Cl_2$ + 1 % TFA): $\lambda_{max}$ (lg $\epsilon$) = 512 nm (5,841), 694 (4,608).

$C_{44}H_{54}N_4$ Ber. 638.4348 Gef. 638.4362 (MS)

| | | | |
|---|---|---|---|
| Ber. | C 82,71 | H 8,52 | N 8,77 |
| Gef. | C 82,80 | H 8,50 | N 8,45 |

Beispiel 8

100 mg (0,14 mmol) des Biladiensalzes aus Beispiel 1 und 1,49 g (14,0 mmol) Benzaldehyd wurden in 40 ml Methanol gelöst und tropfenweise mit 0,2 ml 62 gew.-%iger Bromwasserstoffsäure versetzt. Anschließend erhitzte man das Reaktionsgemisch 3 Stunden unter Rückfluß. Nach raschem Abkühlen auf 20 ° C wurde mit einer Lösung von 63,0 mg (0,28 mmol) Dichlordicyanochinon in 5 ml Dichlormethan versetzt und 1 Stunde bei Raumtemperatur gerührt. Man goß auf 100 ml Dichlormethan und neutralisierte mit gesättigter Natriumhydrogencarbonatlösung. Die wäßrige Phase wurde abgetrennt und mit Dichlormethan extrahiert, die vereinigten organischen Phasen mit Wasser gewaschen und über Magnesiumsulfat getrocknet. Nach weitgehendem Abdampfen des Lösungsmittels reinigte man durch zweimalige Säulenchromatographie an Kieselgel (Laufmittelsystem Dichlormethan/Methanol 25:1 v/v). Das Porphyrin der Formel

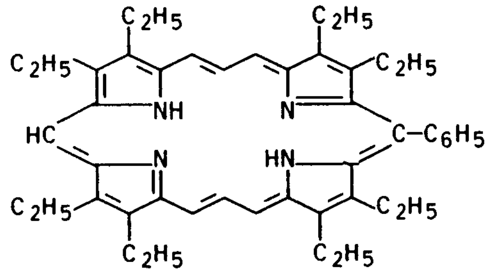

fiel beim Einrotieren der grünen Eluatfraktion als blauer, metallisch glänzender Farblack an. Durch Umkristallisieren aus Chloroform/Ether erhielt man dunkelgrüne, feine Nadeln.
Ausb.: 52,3 mg (56 %)
Schm.: Zers. ohne Schmelzen
$^1$H NMR (300 MHz, $CDCl_3$ + 1 % TFA): δ = -7,94 (t, J = 13,5 Hz, 2 H, Trimethin-Brücken-CH = CHCH), -5,25 und -4,35 (2 br, s, je 2 H, NH), 0,68 (t, J = 7,5 Hz, 6 H, $CH_2CH_3$), 1,66 (t, J = 7,5 Hz, 6 H, $CH_2CH_3$), 2,20 (t, J = 7,5 Hz, 12 H, $CH_2CH_3$), 2,89 (q, J = 7,5 Hz, 4 H, $CH_2CH_3$), 4,16 (q, J = 7,5 Hz, 4 H, $CH_2CH_3$), 4,56 (q, J = 7,5 Hz, 8 H, $CH_2CH_3$), 8,05 (m, 3 H, Phenyl-H, meta und para zum Porphyrin), 8,74 (d, J = 6,9 Hz, 2 H, Phenyl-H, ortho zum Porphyrin), 11,07 (s, 1 H, Monomethin-Brücken-CH), 12,17 und 12,24 (2 d, J = 13,5 Hz, je 2 H, Trimethin-Brücken-CH = CHCH).
MS (EI, 70 eV): m/z (%) = 662 (52) [$M^+$], 647 (22) [$M^+$ - $CH_3$], 633 (22) [$M^+$ -$C_2H_5$], 603 (20) [$M^+$ - $C_2H_5$ - 2 $CH_3$], 588 (12) [$M^+$ - $C_2H_5$ - 3 $CH_3$].
IR (KBr): ν = 3034 $cm^{-1}$ (w, =CH), 2963, 2930 und 2870 (m, CH), 1660 und 1600 (w, konjug. C = C), 1055, 1013, 953 (s).
UV/VIS ($CH_2Cl_2$): $\lambda_{max}$ (lg ε) = 470 nm (5,359), 601 (4,167).
UV/VIS ($CH_2Cl_2$ + 1 % TFA): $\lambda_{max}$ (lg ε) = 474 nm (5,670), 638 (4,209), 690 (3,991).

| | | | |
|---|---|---|---|
| $C_{46}H_{54}N_4 \cdot 0,12\ CHCl_3$ (677,3) Ber. | C 81,78 | H 8,05 | N 8,27 |
| Gef. | C 81,58 | H 8,16 | N 8,21 |

Beispiel 9

Beispiel 9 wurde analog Beispiel 8 durchgeführt, jedoch wurden anstelle von Benzaldehyd 2,12 g (0,14 mmol) 3-Nitrobenzaldehyd verwendet.
Man erhielt 19,1 mg (19 %) des Porphyrins der Formel

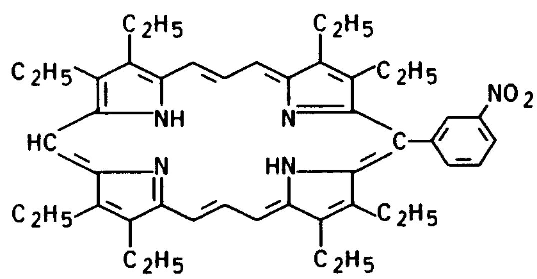

Schm.: Zers. ohne Schmelzen
$^1$H NMR (300 MHz, $CDCl_3$ + 1 % TFA ppm): δ = -8,03 (t, J = 13,5 Hz, 2 H, Trimethin-Brücken-CH = CHCH), -5,25 und -4,35 (2 br, s, je 2 H, NH), 0,72 (t, J = 7,5 Hz, 6 H, $CH_2CH_3$), 1,65 (t, J = 7,5 Hz, 6 H, $CH_2CH_3$), 2,20 (t, J = 7,5 Hz, 12 H, $CH_2CH_3$), 2,85 (q, J = 7,5 Hz, 4 H, $CH_2CH_3$), 4,19 (q, J = 7,5 Hz, 4 H, $CH_2CH_3$), 4,58 (q, J = 7,5 Hz, 8 H, $CH_2CH_3$), 8,27 (t, J = 8,1 Hz, 1 H, Phenyl-H, meta zum Porphyrin), 9,00 (d, J = 9,3 Hz, 1 H, Phenyl-H, para zum Porphyrin), 9,17 (d, J = 7,6 Hz, 1 H, Phenyl-H, ortho zum Porphyrin), 9,67 (s, 1 H, Phenyl-H, ortho zum Porphyrin), 11,15 (s, 1 H, Monomethin-Brücken-CH), 12,24 und 13,20 (2 d, J = 14,0 Hz, je 2 H, Trimethin-Brücken-CH = CHCH).

MS (EI, 70 eV): m/z (%) = 707 (38) [$M^+$], 692 (20) [$M^+$ - $CH_3$], 678 (32) [$M^+$ -$C_2H_5$], 663 (16) [$M^+$ - $C_2H_5$ - $CH_3$].
IR (KBr): $\nu$ = 3035 $cm^{-1}$ (w, =CH), 2963, 2930 und 2870 (m, CH), 1660 und 1600 (w, konjug. C=C), 1530 und 1346 (w, NO), 1055, 1013, 953 (s).
UV/VIS ($CH_2Cl_2$): $\lambda_{max}$ (lg $\epsilon$) = 477 nm (5,682), 639 (4,350), 692 (4,194).
UV/VIS ($CH_2Cl_2$ + 1 % TFA): $\lambda_{max}$ (lg $\epsilon$) = 474 nm (5,712), 639 (4,630), 691 (4,194).

| | | | |
|---|---|---|---|
| $C_{46}H_{53}N_5O_2$ • 0,03 $CHCl_3$ (717,4) Ber. | C 77,06 | H 7,47 | N 9,76 |
| Gef. | C 77,00 | H 7,77 | N 9,70 |

Beispiel 10

Beispiel 10 wurde analog Beispiel 8 durchgeführt, jedoch wurden anstelle von Benzaldehyd 2,30 g (0,14 mmol) 4-Formylbenzoesäuremethylester verwendet.

Man erhielt 20,0 mg (20 %) des Porphyrins der Formel

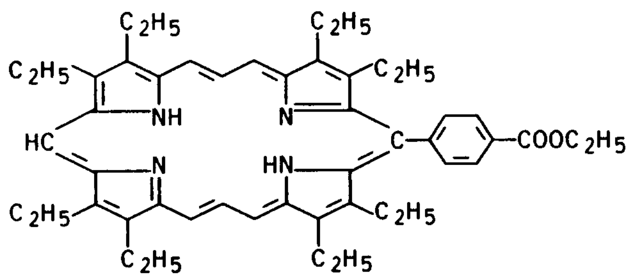

Schm.: Zers. ohne Schmelzen
$^1$H NMR (300 MHz, $CDCl_3$ + 1 % TFA, ppm): $\delta$ = -8,00 (t, J = 13,5 Hz, 2 H, Trimethin-Brücken-CH=CHCH), -5,25 und -4,35 (2 br, s, je 2 H, NH), 0,66 (t, J = 7,5 Hz, 6 H, $CH_2CH_3$), 1,66 (t, J = 7,5 Hz, 6 H, $CH_2CH_3$), 2,19 (t, J = 7,5 Hz, 12 H, $CH_2CH_3$), 2,87 (q, J = 7,5 Hz, 4 H, $CH_2CH_3$), 4,15 (q, J = 7,5 Hz, 4 H, $CH_2CH_3$), 4,28 (s, 3 H, $COOCH_3$), 4,57 (q, J = 7,5 Hz, 8 H, $CH_2CH_3$), 8,72 (d, J = 7,8 Hz, 2 H, Phenyl-H, meta zum Porphyrin), 8,90 (d, J = 8,2 Hz, 2 H, Phenyl-H, ortho zum Porphyrin), 11,12 (s, 1 H, Monomethin-Brücken-CH), 12,21 und 12,28 (2 d, J = 13,5 Hz, je 2 H, Trimethin-Brücken-CH=CHCH).
MS (EI, 70 eV): m/z (%) = 720 (66) [$M^+$], 705 (16) [$M^+$ - $CH_3$], 691 (36) [$M^+$ -$C_2H_5$], 661 (8) [$M^+$ - $C_2H_5$ - 2 $CH_3$], 647 (10) [$M^+$ - $C_2H_5$ - 3 $CH_3$].
IR (KBr): $\nu$ = 3034 $cm^{-1}$ (w, =CH), 2960, 2930 und 2870 (m, CH), 1725 (s, C=O), 1660 und 1590 (w, konjug. C=C), 1055, 1013, 950 (s).
UV/VIS ($CH_2Cl_2$): $\lambda_{max}$ (lg $\epsilon$) = 468 nm (5,390), 601 (4,118), 724 (3,347).
UV/VIS ($CH_2Cl_2$ + 1 % TFA): $\lambda_{max}$ (lg $\epsilon$) = 475 nm (5,694), 639 (4,186), 691 (3,927).

| | | | |
|---|---|---|---|
| $C_{48}H_{54}N_4$ • 0,15 $CHCl_3$ (738,9) Ber. | C 78,28 | H 7,66 | N 7,58 |
| Gef. | C 78,09 | H 7,92 | N 7,54 |

**Patentansprüche**

1. Azaporphyrinderivate der Formel I

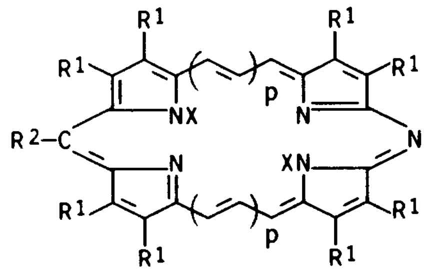

(I),

in der

die Reste $R^1$ und $R^2$ unabhängig voneinander jeweils Wasserstoff, gegebenenfalls substituiertes $C_1$-$C_{20}$-Alkyl, $C_5$-$C_7$-Cycloalkyl oder gegebenenfalls substituiertes Phenyl oder zwei benachbarte Reste $R^1$ zusammen mit den Kohlenstoffatomen, an die sie gebunden sind, einen ungesättigten oder aromatischen 5- bis 7-gliedrigen carbocyclischen Ring,

die Reste X unabhängig voneinander jeweils Wasserstoff oder $C_1$-$C_4$-Alkyl und

p unabhängig voneinander jeweils 1 oder 2 bedeuten.

**2.** Azaporphyrinderivate nach Anspruch 1, dadurch gekennzeichnet, daß die Reste $R^1$ und $R^2$ unabhängig voneinander jeweils Wasserstoff, gegebenenfalls durch Phenyl oder $C_1$-$C_4$-Alkoxycarbonyl substituiertes $C_1$-$C_4$-Alkyl oder Phenyl und die Reste X unabhängig voneinander jeweils Wasserstoff, Methyl oder Ethyl bedeuten.

**3.** Biladiene der Formel II

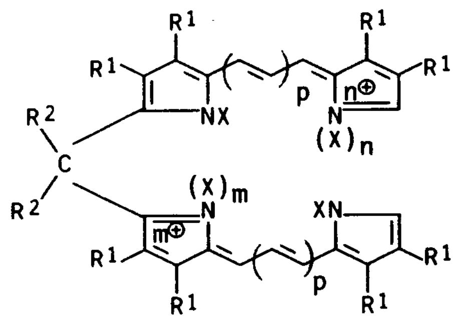

$(m+n)\ An^{\ominus}$ (II),

in der

die Reste $R^1$ und $R^2$ unabhängig voneinander jeweils Wasserstoff, gegebenenfalls substituiertes $C_1$-$C_{20}$-Alkyl, $C_5$-$C_7$-Cycloalkyl oder gegebenenfalls substituiertes Phenyl oder zwei benachbarte Reste $R^1$ zusammen mit den Kohlenstoffatomen, an die sie gebunden sind, einen ungesättigten oder aromatischen 5- bis 7-gliedrigen carbocyclischen Ring,

die Reste X unabhängig voneinander jeweils Wasserstoff oder $C_1$-$C_4$-Alkyl,

m und n unabhängig voneinander jeweils 0 oder 1,
p unabhängig voneinander jeweils 1 oder 2 und
$An^{\ominus}$ ein Anion bedeuten.

**4.** Biladiene nach Anspruch 3, dadurch gekennzeichnet, daß die Reste $R^1$ und $R^2$ unabhängig voneinander jeweils Wasserstoff, gegebenenfalls durch Phenyl oder $C_1$-$C_4$-Alkoxycarbonyl substituiertes $C_1$-$C_4$-Alkyl oder Phenyl und die Reste X unabhängig voneinander jeweils Wasserstoff, Methyl oder Ethyl bedeuten.

**5.** Verwendung der Biladiene gemäß Anspruch 3 zur Herstellung von vinylogen Porphyrinderivaten der

Formel III

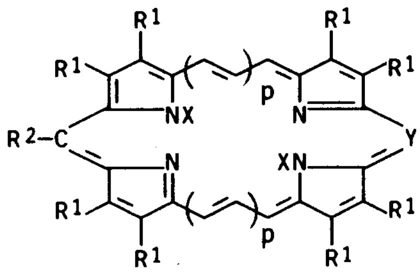

(III),

in der Y für Stickstoff oder den Rest $CR^2$ steht und $R^1$, $R^2$, X und p jeweils die in Anspruch 1 genannte Bedeutung besitzen.

Europäisches Patentamt

# EUROPÄISCHER RECHERCHENBERICHT

Nummer der Anmeldung

**EP 91 11 4905**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D | EP-A-0 264 928 (BASF AG)<br>* Anspruch 1 & US-A-4798891 * | 1 | C 07 D 487/22<br>C 07 D 207/335 //<br>(C 07 D 487/22<br>C 07 D<br>C 07 D 259:00<br>C 07 D 209:00<br>C 07 D<br>C 07 D 209:00<br>C 07 D 209:00<br>C 07 D<br>C 07 D 209:00 ) |
| A,P | EP-A-0 428 013 (BAYER AG)<br>* Beispiel 7 * | 1 | |
| A,D | JUSTUS LIEBIGS ANNALEN DER CHEMIE, no. 9, 1976, Seiten 1637 - 1658; J. ENGEL et al.: "Synthese neuer Biladien-a,c-Derivate und ihre Cyclisierung zu Uroporphyrin-III-, 12-Descarboxyuroporphyrin-III- und ..."<br>* Seiten 1640, 1645 * | 3,5 | |
| A | CHEMICAL ABSTRACTS, vol. 75, no. 11, 13 September 1971 Columbus, Ohio, USA A.W. JOHNSON et al.: "Metal complexes of 1,19-dimethylcorrin" Seite 464; ref. no. 76762U<br>& J. Chem. Soc. D. 1971, [13], 710-11<br>* Zusammenfassung * | 3,5 | |
| A | JOURNAL OF THE CHEMICAL SOCIETY, CHEMICAL COMMUNICATIONS, 1980, Seiten 1124 - 1126; A.R. BATTERSBY et al.: "Biosynthesis of Natural Porphyrins: Further Experiments with Hydroxymethylbilane"<br>* Seite 1125, linke Spalte * | 3,5 | **RECHERCHIERTE SACHGEBIETE (Int. Cl.5)**<br>C 07 D 207/00<br>C 07 D 487/00 |
| A,D | JOURNAL OF ORGANIC CHEMISTRY, vol. 41, no. 17, 1976, Seiten 2826 - 2835; J.B. PAINE III et al.: "Pyrrole Chemistry. The Cyanovinyl Aldehyde Protecting Groups" | | |

Der vorliegende Recherchenbericht wurde für alle Patentansprüche erstellt

| Recherchenort | Abschlußdatum der Recherche | Prüfer |
|---|---|---|
| Berlin | 18 Dezember 91 | HASS C V F |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument